# EUROPEAN PATENT SPECIFICATION

(11) **EP 1 334 976 B1**
(45) Date of publication and mention of the grant of the patent: **08.03.2006**
(21) Application number: 03290239.7
(22) Date of filing: 30.01.2003
(51) Int. Cl.: C07F 9/6568

(54) **Novel phosphine compound, transition metal complex containing said compound as ligand and asymetric synthesis catalyst containing said complex**
Neue Phosphinverbindung, Übergangsmetallkomplex der diese Verbindung als Ligand enthält und Katalysator welcher diesen Komplex enthält für asymmetrische Synthesen
Nouvelles phosphines complexes de métaux de transition contenant ces phosphines comme ligands et catalyseurs pour la synthèse asymétrique contenant ces complexes

(30) Priority: 31.01.2002 JP 2002023568
(43) Date of publication of application: 13.08.2003
(73) Proprietor: Takasago International Corporation, Tokyo 144-8721 (JP)
(72) Inventor: Shimizu, Hideo, c/o Takasago Internation Corp., Hiratsuka-shi, Kanagawa 254-0073 (JP); Saito, Takao, c/o Takasago Internation Corp., Hiratsuka-shi, Kanagawa 254-0073 (JP)
(74) Representative: Uchida, Kenji

(56) References cited:
- DE-A- 19 846 559
- DE-B- 1 282 633
- TSANG, CHI-WING ET AL: "Reactions of Electrophiles with the Phosphaalkene MesP:CH2: Mechanistic Studies of a Catalytic Intramolecular C-H Bond Activation Reaction" ORGANOMETALLICS (2002), 21(6), 1008-1010 , - 18 March 2002 (2002-03-18) XP002235516
- DATABASE CROSSFIRE BEILSTEIN [Online] Beilstein Institut zur Förderung der Chemischen Wissenschaften, Frankfurt am Main, DE; Database accession no. BRN 4935522 XP002235517 & MANN, MILLAR: J. CHEM. SOC., 1951, page 2205
- http://www.faidherbe.org/site/cours/dupuis /sterdyn.htm#binap

## Description

The present invention relates to a novel optically active phosphine compound, a transition metal complex containing the same phosphine compound as a ligand and a transition metal catalyst useful for a variety of asymmetric synthetic reactions.

There have hitherto been many reports on transition metal complexes which can be used for asymmetric synthesis such as asymmetric hydrogenation, asymmetric isomerization, asymmetric hydrosilylation, asymmetric Heck reaction, asymmetric hydroboration and the like. In particular, transition metal complexes in which transition metals such as ruthenium, rhodium, iridium, palladium and the like are coordinated with an optically active phosphine compound provide a good catalyst for asymmetric reaction. Actually, some of such catalysts have been exploited industrially (Asymmetric Catalysis in Organic Synthesis , Ed., R. Noyori, Wiley & Sons, New York, 1994).

Recently, there have been disclosed phospholane type compounds as ligands, which, associated with a transition metal, provide useful catalysts for asymmetric synthesis such as asymmetric hydrogenation, as reported in WO 91/17998 and WO 93/01199.

However, the phospholane type ligands described in the above documents contain an optically active phospholane ring structure, which is prepared from expensive, optically active 1,4-diols. Further, these optically active 1,4-diols are synthesized through specific process and/or means, such as an electrochemical reaction e.g. "Kolbe Reaction". Such a process creates problems for industrial use.

Further yet, the phospholane ligands do not exhibit a satisfactory catalytic activity nor selectivity (diastereo-selectivity, enantio-selectivity), when particular reactions and/or reaction substrates are used. Further improvements of catalysts are therefore strongly desired.

Otherwise, DE-A-19846559 discloses isophosphinedolines of formulae (VII) and (XIII) and Pd, Rh, Ru, Ir and Ni complexes thereof for asymmetric hydrogenations.

Accordingly, an object of the present invention is to provide a novel phosphine compound having excellent properties as to diastereo-selectivity enantio-selectivity, catalytic activity and the like as a ligand of catalysts for asymmetric reactions, in particular, asymmetric hydrogenation reaction. Another object of the invention is to provide an inexpensive preparation method for the novel phosphine compounds.

After extensive studies carried out by the present authors, it has become clear that, when a transition metal complex is coordinated with a novel phosphine compound having a condensed ring of benzene ring and phospholane ring, the transition metal complex thus formed provides a constituent for very active catalysts which catalyze an asymmetric hydrogenation reaction. And it was also found that this transition metal complex has an excellent catalytic activity and enantio-selectivity. The present invention is based on the above finding.

To the above end, there is provided a phosphine compound of the formula (1) or (2): 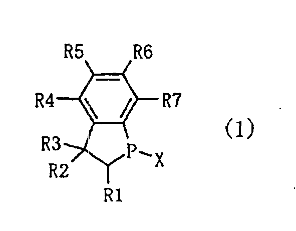
wherein R¹ is a linear or branched alkyl group having 1 to 5 carbon atoms or a cyclohexyl group;
R² and R³ each represent independently either a hydrogen atom or an alkyl group having 1 to 5 carbon atoms;
R⁴ , R⁵ , R⁶ and R⁷ each represent independently a hydrogen atom, a halogen atom, an alkyl group having 1 to 5 carbon atoms, an alkoxy group having 1 to 5 carbon atoms, or a di-alkyl amino group, each alkyl group having 1 to 5 carbon atoms;
X represents a substituted or non-substituted alkyl group, aryl group, aralkyl group, heterocyclic group and alkoxy group, each having 1 to 36 carbon atoms, and an amino group and derivatives thereof; and
wherein a ring or condensed ring may or may not be formed between the members of at least one pair selected from the group consisting of R⁴ and R⁵, R⁵ and R⁶ and R⁶ and R⁷.

The invention also relates to a phosphine compound of the formula (2), 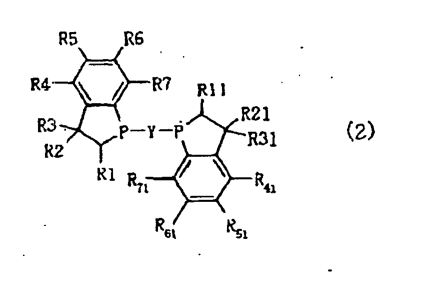
wherein R¹ to R⁷ are as defined above;
R¹¹ is a linear or branched alkyl group having 1 to 5 carbon atoms or a cyclohexyl group;
R²¹ and R³¹ each represent independently either a hydrogen atom or an alkyl group having 1 to 5 carbon atoms;
R⁴¹, R⁵¹, R⁶¹ and R⁷¹ each represent independently a hydrogen atom, a halogen atom, an alkyl group having 1 to 5 carbon atoms, an alkoxy group having 1 to 5 carbon atoms, or a di-alkyl amino group, each alkyl group having 1 to 5 carbon atoms;
Y represents a functional group that may form a stable bond with phosphorous atom; and
wherein a ring or condensed ring may or may not be formed between the members of at least one pair selected from the group consisting of R⁴¹ and R⁵¹, R⁵¹ and R⁶¹, and R⁶¹ and R⁷¹.

In the above formula (2), R¹ and R¹¹ may represent an isopropyl group respectively and Y may represent 1,2-phenylene.

The invention further concerns a transition metal complex comprising a transition metal and, as ligand, a compound defined above.

Preferably, the transition metal comprises at least one metal selected from the group consisting of rhodium, ruthenium, iridium, palladium, and nickel.

Preferably yet, the invention relates to a catalyst for asymmetric syntheses comprising the transition metal complex as defined above.

Suitably, the above asymmetric syntheses comprise the asymmetric hydrogenation.

The invention also relates to a method for preparing a phosphine compound of formula (1) or (2), comprising the step of:
a) reducing 2-halophenylacetic acid using a reducing agent, thereby obtaining 2-(2-halophenyl)ethanol;
b) reacting the 2-(2-halophenyl)ethanol with dialkylcarbamoyl halide in the presence of an amine, thereby obtaining a corresponding carbamate;
c) reacting the carbamate with a reagent which comprises alkyllithium and optically active diamine, then with an alkylating agent, thereby obtaining an alkylated carbamate;
d) reducing the alkylated carbamate, thereby obtaining an optically active secondary alcohol;
e) mesylating or tosylating the hydroxyl group of the secondary alcohol, thereby obtaining a corresponding mesylate or tosylate; and
f) reacting the corresponding mesylate or tosylate successively with a base compound, a functional group that may have a stable bond with a phosphorous atom, and a base compound.

The invention relates in particular to a method for preparing a phosphine compound of formula (2) in which R¹ and R¹¹ represent an isopropyl group, respectively, and Y represents 1,2-phenylene, the method comprising the step of:
a) reacting 2-halogenophenylacetic acid successively with 1,1'-carbonyl diimidazole and alkyl alkylmalonate mono alkaline salt in the presence of a metal halide catalyst, thereby obtaining alkyl 4-(2-halogenophenyl)-2-alkyl-3-oxobutyrate;
b) performing an asymmetric hydrogenation of the alkyl 4-(2-halogenophenyl)-2-alkyl-3-oxobutyrate in the presence of an optically active diphosphine-Ru complex, thereby obtaining an optically active alkyl 4-(2-halogenophenyl)-3-hydroxy-2 -alkylbutyrate;
c) reducing the alkylbutyrate with a reducing agent, thereby obtaining a diol;
d) tosylating or mesylating the primary hydroxyl group of the diol, thereby obtaining a tosylate or mesylate;
e) reducing the tosylate or mesylate with a reducing agent, thereby obtaining an optically active 1-(2-halogenophenyl) -3-alkylbutan-2-ol;
f) mesylating or tosylating the alcohol, thereby obtaining a mesylate or tosylate thereof; and
reacting the mesylate or tosylate successively with a base compound, 1,2-bisphosphinobenzene and a base compound.
   The invention further concerns a use of a phosphine compound of the formula (1) or (2), for preparing a catalyst for asymmetric syntheses.
   The above, and the other objects, features and advantages will be made apparent from the following description of the preferred embodiments, given as non-limiting examples.
   One of the phosphine compounds of the present invention is represented by the following general formula (1):

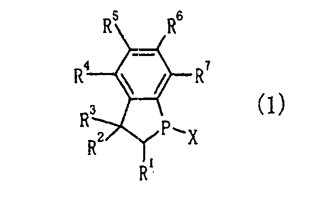

wherein R¹ is a linear or branched alkyl group having 1 to 5 carbon atoms or a cyclohexyl group; R² and R³ represent independently a hydrogen atom or an alkyl group having 1 to 5 carbon atoms; R⁴, R⁵ , R⁶ and R⁷ represent independently a hydrogen atom, a halogen atom, an alkyl group having 1 to 5 carbon atoms, an alkoxy group having 1 to 5 carbon atoms, and a dialkyl amino group, each of both alkyls having 1 to 5 carbon atoms; and X represents functional group that may form a stable bond with phosphorous atom; wherein a ring or condensed ring may or may not be formed between the members of at least one pair selected from the group consisting of R⁴ and R⁵, R⁵ and R⁶, and R⁶ and R⁷.

Examples of R¹ mentioned above include methyl group, ethyl group, n-propyl group, isopropyl group, n-butyl group, sec-butyl group, isobutyl group, tert-butyl group, n-pentyl group, isopentyl group, and neopentyl group.

Examples of R² and R³ mentioned above include methyl group, ethyl group, n-propyl group, isopropyl group. Examples of R⁴, R⁵, R⁶, R⁷ in the above formula include a halogen atom, such as fluorine atom, chlorine atom, bromine atom and iodine atom; an alkyl group having 1 to 5 carbon atoms, such as methyl group, ethyl group, n-propyl group, isopropyl group, n-butyl group, sec-butyl group, isobutyl group, tert-butyl group, n-pentyl group, isopentyl group, neopentyl group; an alkoxy group having 1 to 5 carbon atoms, such as methoxy group , ethoxy group , n-propoxy group , isopropoxy group , tert-butoxy group; a dialkyl amino group each alkyl having 1 to 5 carbon atoms, such as dimethylamino group , diethylamino group , pyrrolidino group , piperidino group.

Examples of ring or condensed ring include a five to seven membered ring such as a benzene ring, cyclopentene ring, cyclohexene ring, cyclopentadiene ring, indene ring and a naphthalene ring, and a heterocycle containing a methylenedioxy group, an ethylenedioxy group or a trimethylenedioxy group.

Specific examples of X comprise a substituted or non-substituted alkyl group, aryl group, aralkyl group, heterocyclic group and alkoxy group, each having 1 to 36 carbon atoms, and amino group and its derivatives. When substituted, the substituent may be alkyl group having 1 to 5 carbon atoms, alkoxy group having 1 to 5 carbon atoms, haloalkyl group having 1 to 5 carbon atoms, halogen atom, amino group, mono alkylamino group and di alkylamino group. Examples of alkyl group and alkoxy group respectively having 1 to 5 carbon atoms are the same as those exemplified above.

Preferred examples of X include a substituted or non-substituted phenyl group, such as phenyl group, 3-methoxyphenyl group, 3,4-methylenedioxyphenyl group, 1-naphthyl group, 2-naphthyl group, 3,5-di(tert-butyl)-4-methoxy phenyl group, 2-(diphenylphosphino) phenyl group, 2-(di(4-tolyl) phosphino) phenyl group, 2-(di(3,5-xylyl)phosphino) phenyl group, 2-(bis(3,5-di(tert-butyl)-4-methoxyphenyl)phosphino) phenyl group, 2-(di(1-naphthyl)phosphino) phenyl group, 2-(di(2-naphthyl) phosphino) phenyl group, 6-methoxy-2-(diphenylphosphino) phenyl group and 5,6-methylenedioxy-2-(diphenylphosphino)phenyl group.

Another compound of the present invention is represented by the following general formula (2): 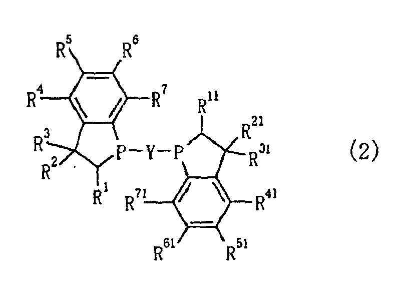
wherein R¹ - R⁷ are as defined above; R¹¹ is a linear or branched alkyl group having 1 to 5 carbon atoms or a cyclohexyl group; R²¹ and R³¹ represent independently a hydrogen atom or an alkyl group having 1 to 5 carbon atoms; R⁴¹, R⁵¹, R⁶¹ and R⁷¹ represent independently a hydrogen atom, a halogen atom, an alkyl group having 1 to 5 carbon atoms, an alkoxy group having 1 to 5 carbon atoms, and a dialkyl amino group, each of both alkyls having 1 to 5 carbon atoms; Y represents a functional group that may form a stable bond with phosphorous atom; wherein a ring or condensed ring may or may not be formed between the members of at least one pair selected from the group consisting of R⁴¹ and R⁵¹, R⁵¹ and R⁶¹, and R⁶¹ and R⁷¹.

Examples of R¹¹ mentioned above include methyl group, ethyl group, n-propyl group, isopropyl group, n-butyl group, sec-butyl group, isobutyl group, tert-butyl group, n-pentyl group, isopentyl group and neopentyl group.

Examples of R²¹ and R³¹ include methyl group, ethyl group, n-propyl group, isopropyl group.

Examples of R⁴¹, R⁵¹, R⁶¹, R⁷¹ include a halogen atom, such as fluorine atom, chlorine atom ,bromine atom and iodine atom; an alkyl group having 1 to 5 carbon atoms, such as methyl group , ethyl group , n-propyl group , isopropyl group , n-butyl group , sec-butyl group , isobutyl group , tert-butyl group , n-pentyl group , isopentyl group , neopentyl group are mentioned; an alkoxy group having 1 to 5 carbon atoms, such as methoxy group , ethoxy group , n-propoxy group , isopropoxy group , tert-butoxy group are mentioned; a dialkyl amino group each alkyl having 1 to 5 carbon atoms, such as dimethylamino group, diethylamino group, pyrrolidino group and piperidino group.

Examples of ring or condensed ring include a five to seven membered ring such as a benzene ring, cyclopentene ring, cyclohexene ring, cyclopentadiene ring, indene ring and a naphthalene ring, and a heterocycle containing a methylenedioxy group, an ethylenedioxy group or a trimethylenedioxy group.

Examples of Y include a substituted or non-substituted alkylene group, arylene group and divalent heterocyclic group, respectively having 1 to 36 carbon atoms; groups -O-R¹³-O- , -O-R¹⁴- , -NR²³⁻R¹⁵⁻, -NR²⁴-R¹⁶-NR²⁵-, and -NR²⁶-R¹⁷-O- , in which R¹³-R¹⁷ represent independently a substituted or non-substituted alkylene group and arylene group respectively having 1 to 36 carbon atoms, whereas R²³-R²⁶ represent independently a hydrogen atom and a substituted or non-substituted alkyl group having 1 to 5 carbon atoms; and a divalent group having a substituted or non-substituted alkylene structure and a substituted or non-substituted arylene structure.

When substituted, examples of substituents include an alkyl group having 1 to 5 carbon atoms, an alkoxy group having 1 to 5 carbon atoms, a haloalkyl group having 1 to 5 carbon atoms, a halogen atom, an amino group, a monoalkylamino group and a dialkylamino group.

Examples of divalent group having a heterocyclic structure include pyridine-2, 6-diyl (structure A) and phosphabenzene-2, 6-diyl (structure B).

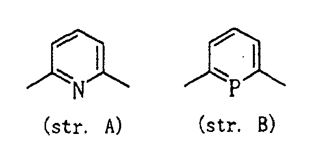

Examples of divalent group having alkylene and arylene structures include toluene-α,2-diyl group (structure C), ethylbenzene-B,2-diyl (structure D) , o-xylene-α,α'-diyl (structure E).

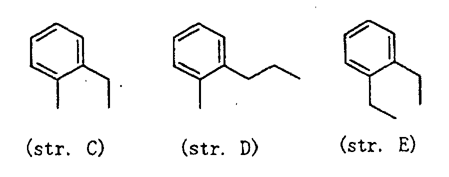

Amongst them, preferred example of Y include a substituted or non-substituted phenylene or alkylene group. More preferred examples include 1,2-phenylene group, 3-methoxy-1,2-phenylene group, 3,4-methylenedioxy-1,2-phenylene group, methylene group, ethylene group, trimethylene group, tetramethylene group, pentamethylene group and hexamethylene group. Most preferred examples include 1,2-phenylene group , methylene group and ethylene group.

The compounds of the present invention described above also include racemic compounds, meso form compounds and optically active compounds.

Amongst the aforementioned compounds, preferred are compounds in which R², R³, R²¹, R³¹ represent hydrogen atom.

Further yet, compounds in which R⁴, R⁵, R⁶, R⁷, R⁴¹, R⁵¹, R⁶¹, R⁷¹ represent hydrogen atom are also preferred. Most preferred is a compound in which all the above substituents represent hydrogen.

Typically, R¹ and R¹¹ in the formula (2) may be an isopropyl group and Y may be a benzene group.

A process for preparing the compounds according to the present invention will be described hereunder.

As illustrated in SCHEME 1, the method for preparing a phosphine compound of formula (1) or (2) typically comprises the step of:
a) reducing 2-halophenylacetic acid using a reducing agent, thereby obtaining 2-(2-halophenyl)ethanol;
b) reacting 2-(2-halophenyl)ethanol with dialkylcarbamoyl halide in the presence of an amine, thereby obtaining a corresponding carbamate;
c) reacting the carbamate with a reagent which consists of alkyllithium and optically active diamine, then with an alkylating agent, thereby obtaining an alkylated carbamate;
d) reducing the alkylated carbamate, thereby obtaining an optically active secondary alcohol;
e) mesylating or tosylating the hydroxyl group of the secondary alcohol, thereby obtaining a corresponding mesylate or tosylate; and
f) reacting the mesylate or tosylate successively with a base compound, a bisphosphine compound and a base compound.

In the above method, examples of base compounds include alkyllithium, metal hydride, metal amide, metal hydroxide.

Further, examples of halogen atom include fluorine atom, chlorine atom, bromine atom and iodine atom.

Examples of reducing agents include any metal hydrides which have reducing ability, e.g. lithium aluminium hydride, sodium borohydride and diisobutylaluminium hydride (DIBAL).

Examples of alkyl group of dialkylcarbamoyl chloride include methyl group, ethyl group, n-propyl group, iso-butyl group, n-butyl group, iso-butyl group and tert-butyl group.

Examples of amine include triethylamine, diisopropylethylamine, pyridine and 4-(dimethylamino)pyridine.

Examples of optically active diamine include any diamine compound which has chirality, e.g. (-)-sparteine.

Examples of alkyllithium include methyllithium, ethyllithium, n-propyllithium, iso-propyllithium, n-butyllithium, sec-buthyllithium, tert-buthyllithium and phenyllithium.

Examples of alkylating agent include alkyl iodode, alkyl bromide, alkyl chloride, alkyl sulfate, alkyl tosylate and alkyl mesylate, in which alkyl group of alkylating agent is a linear or branched alkyl group having 1 to 6 carbon atoms.

Examples of metal hydride include lithium hydride, sodium hydride and potassium hydride.

Examples of metal amide include lithium diethylamide, lithium diisopropyl amide, lithium amide and sodium amide.

Examples of metal hydroxide include lithium hydroxide, sodium hydroxide and potassium hydroxide.

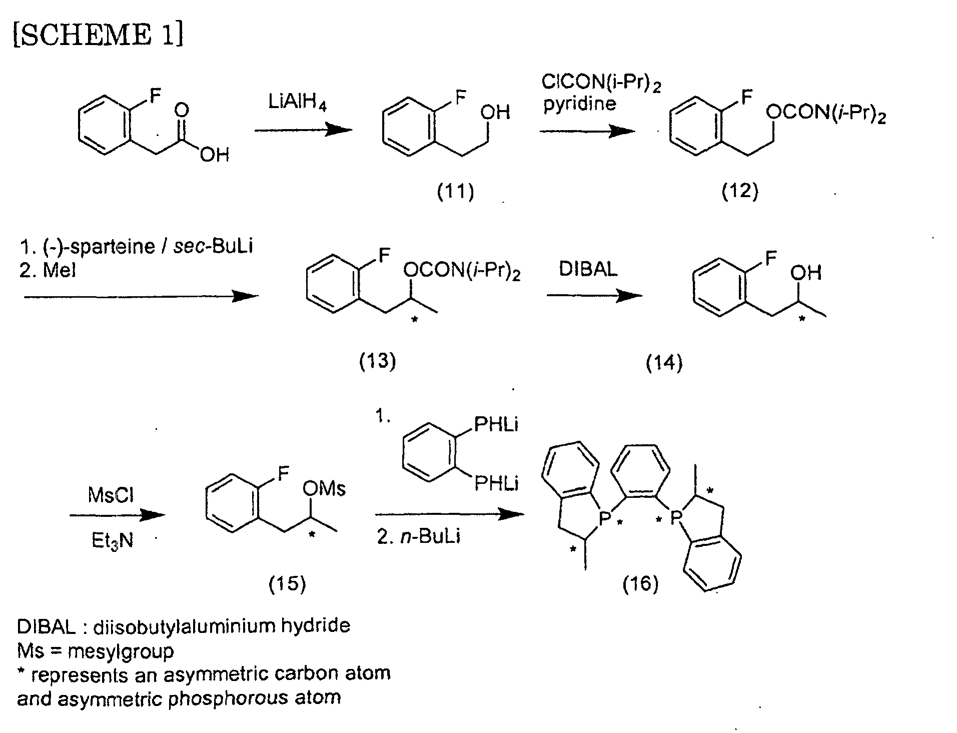

Alternatively, the method for preparing a phosphine compound of formula (1) or (2) typically comprises the steps of:
reacting 2-halogenophenylacetic acid successively with 1,1'-carbonyl diimidazole and alkyl alkylmalonate mono alkaline salt in the presence of a metal halide catalyst, thereby obtaining alkyl 4-(2-halogenophenyl)-2-alkyl-3-oxobutyrate;
performing an asymmetric hydrogenation of the formed compound in the presence of an optically active diphosphine-Ru complex, thereby obtaining an optically active alkyl 4-(2-halogenophenyl)-3-hydroxy-2 -alkylbutyrate;
reducing the alkylbutyrate with a reducing agent, thereby obtaining a diol;
tosylating or mesylating the primary hydroxyl group of the diol, thereby obtaining a tosylate or mesylate;
reducing the tosylate or mesylate with a reducing agent, thereby obtaining an optically active 1-(2-halogenophenyl) -3-alkylbutan-2-ol;
mesylating or tosylating the alcohol, thereby obtaining a mesylate or tosylate thereof; and
reacting the mesylate or tosylate successively with a base compound, a bisphosphine compound and a base compound.

In the above method, the base compound may be alkyllithium, metal hydride, metal amide, metal hydroxide.

Further, examples of halogen atom used include fluorine atom, chlorine atom, bromine atom, iodine atom; examples of alkyl group of alkyl methyl malonate include methyl group, ethyl group, etc.: examples of alkaline atom include sodium, potassium, magnesium, etc.; examples of metal halides include magnesium chloride, magnesium bromide, magnesium iodide, zinc chloride, zinc bromide, etc.

Further, examples of optically active diphosphines include diphosphines disclosed e.g. in Handbook of Enantioselective Catalysis Vol. II , VCH, Synlett, 2001, 1055 and J.Am.Chem.Soc, 1993, 115, 10125. Those diphosphines include, in particular, BINAP, Tol-BINAP, DM-BINAP, H₈-BINAP, DM-H₈-BINAP, SEGPHOS, Tol-SEGPHOS, DM-SEGPHOS, DMM-SEGPHOS, DTBM - SEGPHOS, BIPHEMP, MeO - BIPHEMP, DIPAMP, DIOP, CHIRAPHOS, SkewPHOS, DuPHOS, BPE, PHANEPHOS, JOSIPHOS, etc.

Examples of optically active diphosphine-Ru complexes include Ru(OAc)₂(L), Ru(OCOCF₃)₂(L), Ru₂Cl₄(L)₂NEt₃ , [{RuCl(L)}₂(µ-Cl)₃][Me₂NH₂], [{RuBr(L)}₂(µ-Br)₃][Me₂NH₂], [{RuI(L)}₂(µ-I)₃][Me₂NH₂], [{RuCl(L)}₂(µ-Cl)₃][Et₂NH₂], [{RuBr(L)}₂(µ-Br)₃][Et₂NH₂], [{RuI(L)}₂(µ-I)₃][Et₂NH₂], RuCl₂(L), RuBr₂(L), RuI₂(L), RuCl₂(L)(pyridine)₂, RuBr₂(L) (pyridine)₂, RuI₂(L)(pyridine)₂, [RuCl(benzene)(L)]Cl, [RuBr(benzene)(L)]Br, [RuI(benzene)(L)]I, [RuCl(p-cymene)(L)]Cl, [RuBr(p-cymene)(L)] Br, [RuI(p-cymene)(L)]I, [Ru(L)](OTf)₂, [Ru(L)](BF₄)₂, [Ru(L)](ClO₄)₂, [Ru(L)](SbF₆)₂, [Ru(L)](PF₆)₂, [Ru(L)](BPh₄)₂, etc.

In the above formula, L means optically active diphosphine compounds.

Examples of reducing agents include any metal hydrides which have reducing ability, e.g. lithium aluminium hydride, sodium borohydride.

Examples of alkyllithium include methyllithium, ethyllithium, n-propyllithium, iso-propyllithium, n-butyllithium, sec-buthyllithium, tert-buthyllithium, phenyllithium.

Examples of metal hydride include lithium hydride, sodium hydride, potassium hydride.

Examples of metal amide include lithium diethylamide, lithium diisopropyl amide, lithium amide, sodium amide.

Examples of metal hydroxide include lithium hydroxide, sodium hydroxide, potassium hydroxide.

In the following, a representative embodiment of the inventive process is described with reference to an optically active and (+)-form compound, (+)-1,2-bis(2-isopropyl-2,3-dihydro-1H-phosphindol-1-yl) benzene (hereafter sometimes referred to as "(+)-iPr-BeePHOS") of the formula (+)-(3): 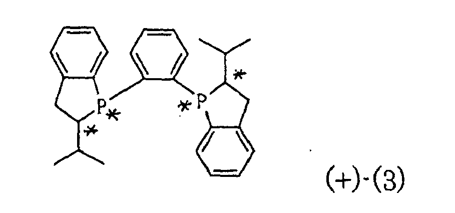 (the sign * represents an asymmetric carbon or phosphorous atom).

However, the present invention is not particularly limited to the above example.

First, 2-fluorophenylacetic acid (4) is treated with 1,1'-carbonyl diimidazole (CDI), and then reacted with ethyl methylmalonate mono potassium salt in the presence of magnesium chloride, to obtain ethyl 4-(2-fluorophenyl)-2-methyl -3-oxobutyrate (5).

The compound (5) thus obtained is asymmetrically hydrogenated using ((R)-SEGPHOS™)-Ru complex, e.g. in 3 MPa of atmospheric hydrogen pressure, to obtain optically active ethyl 4-(2-fluorophenyl) -3-hydroxy-2-methylbutyrate (6) as a diastereomer mixture.

In the above formula, SEGPHOS means {(5,6),(5',6')-bis(methylenedioxy)biphenyl-2,2'-diyl} bis(diphenylphosphine). Then , compound (6) is reduced with lithium aluminum hydride to obtain diol (7).

The primary hydroxyl group of the diol (7) is then tosylated, and tosylate (8) thus obtained is reduced with lithium aluminum hydride to give (+)-1-(2-fluorophenyl)-3-methylbutan-2-ol {(+)-(9)}. Thereafter, the alcohol {(+)-(9)} is transformed to mesylate {(+)-(10)}. Then, 1,2-bisphosphinobenzene is treated successively with n-butyl lithium, the above mesylate {(+)-(10)} and n-butyl lithium, to give (+)-1,2-bis(2-isopropyl-2,3-dihydro-1H-phosphindol-1-yl) benzene, i.e. compound (+)-(3), as a single diastereomer.

Accordingly, the compound (+)-(3) is produced by the process shown in the following reaction scheme 2.

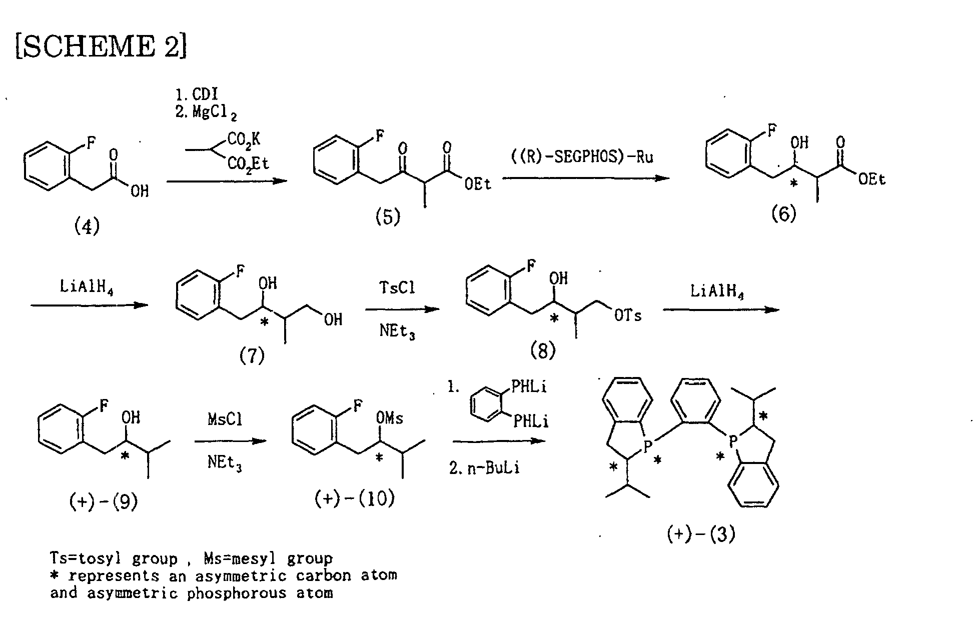

In addition, compound (-)-(3) can be obtained by using (S)-SEGPHOS™ instead of (R)-SEGPHOS™. Further, other optically active phosphine ligands may be used instead of SEGPHOS™. Furthermore , other generally known transition metal complexes may be used for asymmetric synthesis.

Among the above compounds, the optically active forms of racemic compound (3), in particular, can be obtained by optical resolution of enantiomers by performing a High Performance Liquid Chromatography (HPLC) in an optically active column. Optically active compound (3) can also be obtained in a following manner: racemic compound (3) is first oxidized to corresponding diphosphine oxide, the enantiomers of which are then optically separated with an optically active column, and reduced with a silane compound.

Likewise, optically active alcohol (9) can be obtained by optically resolving racemic alcohol (9) by enzyme . The phosphine compounds of the present invention represented by the general formula (1) are prepared by using corresponding primary phosphine instead of 1,2-bisphosphinobenzene in [SCHEME 2].

Examples of corresponding primary phosphines include: phenylphosphine, 3-methoxyphenylphosphine, 3,4-methylenedioxy phenyl phosphine, 1-naphthylphosphine, 2-naphthylphosphine, 3,5-di(tert-butyl)-4 -methoxyphenylphosphine, 2-(diphenylphosphino) phenylphosphine, 2-(di (4-tolyl)phosphino)phenylphosphine, 2-(di(3,5-xylyl)phosphino)phenyl phosphine, 2-(bis(3,5-di(tert-butyl) -4-methoxyphenyl)phosphino)phenyl phosphine, 2-(di(1-naphthyl)phosphino)phenylphosphine, 2-(di(2-naphthyl) phosphino)phenylphosphine, 6-methoxy-2-(diphenylphosphino) phenyl phosphine and 5,6-methylenedioxy-,2-(diphenylphosphino) phenyl phosphine. The phosphine compounds of the present invention represented by the general formula (2) are prepared by using corresponding primary diphosphine instead of 1,2-bisphosphinobenzene in [SCHEME 2].

Examples of corresponding primary diphosphines include: 3-Methoxy-1,2-bisphosphinobenzene,3,4-methylenedioxy-1,2-bisp hosphinobenzene, 1,1-bisphosphinomethane, 1,2-bisphosphinoethane, 1,3-bis phosphinopropane, 1,4-bisphosphinobutane and 1,5-bisphosphino pentane.

Likewise, the phosphine compounds represented by the general formula (1) or (2), i.e. excluding compound (3), are prepared by using a corresponding alcohol instead of compound (9) in [SCHEME 2].

Examples of corresponding alcohols include: 1-(2-fluorophenyl)propan-2-ol, 1-(2-fluorophenyl)butan-2-ol, 3-(2-fluorophenyl)butan-2-ol, 3-(2-fluorophenyl)-3-methylbutan-2-ol, 2-(2-fluoro phenyl)pentan-3-ol, 2-(2-fluorophenyl) -4-methyl pentan-3-ol and 2-(2-fluorophenyl)-2,4-dimethylpentan -3-ol.

Among the compounds of the invention, optically active phosphine compounds of the formula (1) or (2), in particular, are useful as ligands for transition metal complexes.

One of the transition metal complexes containing such ligands is represented by the general formula (31):

MmLnWpUq (31)

wherein M is at least one transition metal selected from the group consisting of Ir , Rh , Ru, Pd and Ni; L represents one or two molecule(s) of phosphine compound of the general formula (1), or one molecule of phosphine compound of the general formula (2); and
in case where M is Ir or Rh,
m, n and p are respectively 2 and q is 0, when W is Cl, Br or I;
in case where M is Ru,
(i) U represents a trialkylamino group, m and n are respectively 2, p is 4, and q is 1, when W is Cl, Br or I,
(ii) U represents a pyridyl group or a pyridyl group the ring of which is substituted, m and n are respectively 1, and p and q are respectively 2, when W is Cl, Br, or I,
(iii) m and n are respectively 1, p is 2 and q is 0, when W is a carboxlate group,
(iv) m and n are respectively 1, p is 2 and q is 0, when W is Cl, Br or I, or
(v) U represents a dialkylammonium ion, m and n are respectively 2, p is 5 and q is 1, when W is Cl, Br or I;
   in case where M is Pd,
(i) m and n are respectively 1, p is 2 and q is 0, when W is Cl, Br or I, or
(ii) m, n and p are respectively 2 and q=0, when W is allyl group; and

in case where M is Ni,
m and n are respectively 1, p is 2 and q is 0, when W is Cl, Br or I.
In the above complex, examples of substituents for pyridyl
ring include an alkyl group having 1 to 3 carbon atoms, a halogen atom or the like. The carboxylate group includes, for instance, CH₃COO, CH₃COCH₂COO and the like.

Further, one of the transition metal complexes of the invention is represented by the general formula (32):

[MmLnWpUq]Zs (32)

wherein M is at least one transition metal selected from the group consisting of Ir, Rh, Ru, Pd and Ni; L represents an optically active compound of one or two molecule(s) of inventive compound of formula (1), or one molecule of inventive compound of formula (2); and
in case where M is Ir or Rh, ,
Z is BF₄, ClO₄, OTf, PF₆, SbF₆ or BPh₄, and either m, n, p and s are respectively 1 and q is 0, or m is 1, n is 2, p and q are respectively 0 and s is 1, when W is 1,5-cyclooctadiene or norbornadiene;
in case where M is Ru,
(i) U represents a neutral ligand e.g. aromatic compound and olefin compound, Z is Cl, Br, I, I₃ or sulfonate, and m, n, p, s and q are respectively 1, when W is Cl, Br or I, or
(ii) m and n are respectively 1, p and q are respectively 0 and s is 2, when Z is BF₄, ClO₄, OTf, PF₆, SbF₆ or BPh₄;
   in case where M is Pd or Ni,
(i) m and n are respectively 1, p and q are respectively 0 and s is 2, when Z is BF₄, ClO₄, OTf, PF₆, SbF₆ or BPh₄.

In the above formula, Tf signifies group SO₂CF₃. In the above complexes, the aromatic compound as a neutral ligand includes, for instance, benzene, p-cymene and the like, and examples of olefin compounds include 1,5-cyclooctadiene, norbornadiene or the like.
These transition metal complexes can be produced by a known method.

Further, in the symbols used in the formulae shown in the following transition metal complexes, "L" represents an optically active compound of one or two molecule(s) of inventive compound of formula (1), or one molecule of inventive compound of formula (2). "cod", "nbd", "Ph" and "Ac" represent, respectively, 1,5-cyclooctadiene, norbornadiene, phenyl group, and acetyl group.

### Rhodium Complex:

Typically, a rhodium complex may be synthesized by reacting bis(cod)rhodium(I) tetrafluoroborate salt with phospholane compound (1) or (2) of the present invention (hereinafter referred to as BeePHOS), according to the method described in "4^{th} edition Jikken Kagaku koza (Lectures on Experimental Chemistry)", vol. 18, Organic Metal Complexes, 1991, published by Maruzen, pp.339-344, edited by the Chemical Society of Japan.

Examples of specific examples of rhodium complex include: [Rh(L)Cl]₂ , [Rh(L)Br]₂, [Rh(L)I]₂ , [Rh(cod)(L)]OTf, [Rh(cod)(L)]BF₄, [Rh(cod)(L)]ClO₄, [Rh(cod)(L)]SbF₆, [Rh(cod)(L)]PF₆, [Rh(cod)(L)]BPh₄, [Rh(nbd)(L)]OTf, [Rh(nbd)(L)]BF₄, [Rh(nbd)(L)]Cl0₄, [Rh(cod)(L)]SbF₆, [Rh(nbd)(L)]PF₆, [Rh(nbd)(L)]BPh₄, [Rh(L)₂]ClO₄, [Rh(L)₂]OTf, [Rh(L)₂]BF₄, [Rh(L)₂]PF₆, [Rh(L)2]SbF₆ and [Rh(L)2]BPh₄.

### Ruthenium Complex:

Typically, a ruthenium complex may be prepared by heating [Ru(cod)Cl₂]ₙ and BeePHOS under reflux in the presence of triethylamine in toluene solvent as described in a prior art (T. Ikariya et al, J.Chem.Soc.,Chem.Commun.,922, 1988). Moreover it may also be prepared by heating [Ru(p-cymene)I₂]₂ and BeePHOS under stirring in methylene chloride and ethanol, according to a method described in a prior art (K. Mashima et al., J. Chem. Soc., Chem. Commun., 1208, 1989).

Examples of ruthenium complex include:
Ru(OAc)₂(L), Ru(OCOCF₃)₂(L), Ru₂Cl₄(L)₂NEt₃, [{RuCl(L)}₂( µ -Cl)₃] [Me₂NH₂], [{RuBr(L)}₂( µ -Br)₃][Me₂NH₂], [{RuI(L)}₂( µ -I)₃][Me₂NH₂], [{RuCl(L)}₂( µ -Cl)₃][Et₂NH₂], [{RuBr(L)}₂( µ -Br)₃][Et₂NH₂], [{RuI(L)}₂ (µ-I)₃][Et₂NH₂], RuCl₂(L), RuBr₂(L), RuI₂(L), RuCl₂(L)(pyridine)₂, RuBr₂(L)(pyridine)₂ , RuI₂(L)(pyridine)₂ , [RuCl(benzene)(L)]Cl, [RuBr(benzene)(L)]Br, [RuI(benzene)(L)]I , [RuCl(p-cymene)(L)]Cl , [RuBr(p-cymene)(L)]Br, [RuI(p-cymene)(L)]I, [Ru(L)](OTf)₂, [Ru(L)](BF₄)₂, [Ru(L)](ClO₄)₂, [Ru(L)](SbF₆)₂ and [Ru(L)](BF₄)_{2.}

### Iridium Complex:

Suitably, an iridium complex may be prepared by reacting BeePHOS and iridium compound, such as [Ir(cod)(CH₃CN)₂]BF₄ in tetrahydrofuran, according to the method described in a prior art (K. Mashima et al, J. Organomet. Chem., 428, 213, 1992).

### Examples of iridium complex include:

[Ir(L)Cl]₂ , [Ir(L)Br]₂ , [Ir(L)I]₂ , [Ir(cod)(L)]OTf , [Ir(cod)(L)]BF₄, [Ir(cod)(L)]ClO₄, [Ir(cod)(L)]SbF₆, [Ir(cod)(L)]PF₆, [Ir(cod)(L)]BPh₄, [Ir(nbd)(L)]OTf, [Ir(nbd)(L)]BF₄, [Ir(nbd)(L)]ClO₄, [Ir(cod)(L)]SbF₆, [Ir(nbd)(L)]PF₆, [Ir(nbd)(L)]BPh₄, IrCl(cod)(CO)(L), IrBr(cod)(CO)(L), IrI(cod)(CO)(L), [Ir(L)₂]ClO₄, [Ir(L)₂]OTf, [Ir(L)₂]BF₄, [Ir(L)₂]PF₆, [Ir(L)₂]SbF₆ and [Ir(L)₂]BPh₄.

### Palladium Complex:

Typically, a palladium complex may be prepared by reacting BeePHOS and π-allylpalladium compound according to a method described in a prior art (Y. Uozumi et al., J. Am. Chem. Soc., 113, 9887, 1991).

Examples of palladium complex include: PdCl₂(L), PdBr₂(L), PdI₂(L), Pd(OAc)₂(L), Pd(OCOCF₃)₂(L), [(π -allyl) Pd(L)]Cl, [( π -allyl)Pd(L)]Br, [( π -allyl)Pd(L)]I, [( π -allyl)Pd(L)]OTf, [(π-allyl)Pd(L)]BF₄, [(π-allyl)Pd(L)]ClO₄, [(π -allyl)Fd(L)]SbF₆, [(π-allyl)Pd(L)]PF₆, [(π-allyl)Pd(L)]BPh₄, [Pd(L)](OTf)₂, [Pd(L)](BPh₄)₂, [Pd(L)](PF₆)₂, [Pd(L)](ClO₄)₂, [Pd(L)](BF₄)₂, [Pd(L)](SbF₆)₂, PhCH₂Pd(L)Cl, PhCH₂Pd(L)Br, PhCH₂Pd(L)I, PhFdCl(L), PhPdBr(L) and PhPdI(L).

### Nickel Complex:

Typically, a nickel complex may be prepared by a method described in "4^{th} edition Jikken Kagaku Koza (Lectures on Experimental Chemistry)", vol. 18 , Organic Metal Complexes , 1991 , published by Maruzen, p. 376, edited by the Chemical Society of Japan, or by dissolving BeePHOS and nickel chloride in a mixed solvent of 2-propanol and methanol and heating them under stirring, according to a method described in a prior art (Y Uozumi et al., J. Am. Chem. Soc., 113, 9887, 1991).

Examples of nickel complex include:
NiCl₂(L), NiBr₂(L) and NiI₂(L)

A transition metal complex containing an optically active phosphine compound of the invention as a ligand is an efficient catalyst for asymmetric synthesis, e.g. asymmetric hydrogenation, asymmetric isomerization and asymmetric hydroformylation. In addition , the racemic form of BeePHOS is useful as an intermediate product for corresponding optically active compounds. As a catalyst, the complex may be used without prior purification, or after its purity has been raised.

As it can be understood from the foregoing, the novel phosphine compounds of the present invention are particularly useful as a ligand for a transition metal complex, which catalyzes e.g. asymmetric hydrogenation. These inventive novel phosphine compounds may be produced by a relatively economical method.

In addition, asymmetrically hydrogenated products can be obtained in high yield and high optical purity by using the catalysts of the invention. The above catalysts are thus very useful from the view point of industrial production.

The following Examples and Use Example will illustrate the present invention in more detail.

### Examples

Apparatuses used for determining physical properties in the following Examples are as follows:
Nuclear magnetic resonance: ¹H-NMR Bruker AM400 (400MHz);
³¹P-NMR Bruker AM400 (202MHz).
Optical rotation: Nihon Bunkoh Co., Ltd. DIP-4.
Gas chromatography GLC: Hewlett Packard 5890-II.
High-performance liquid chromatography HPLC: Hewlett Packard
HP1100.
Mass spectrometry (MS): M-80B manufactured by Hitachi.

### Example 1: Synthesis of (+)-1,2-bis(2-isopropyl-2,3-dihydro phosphindol -1-yl)benzene, referred to as (+)-iPr-BeePHOS.

### (a) Synthesis of ethyl 4-(2-fluorophenyl)-2-methyl-3-oxobutyrate.

Under a nitrogen atmosphere, 20 g (129 mmol) of 2-fluorophenylacetic acid was dissolved in 30 ml of acetonitrile, and 23.0 g (142 mmol) of 1,1'-carbonyl diimidazole (CDI) was added thereto at room temperature. After the mixture was stirred for one hour, the obtained reaction solution was dropped into a suspension comprising 33.3 g (180 mmol) of potassium monoethyl methylmalonate, 14.7 g (155 mmol) of magnesium chloride and 60 ml of acetonitrile, and then stirred overnight at 45 °C. 200 ml of 1N-HCl was added to the reaction solution, and the whole solution was extracted 3 times with 200 ml each of ethyl acetate. The combined organic layer was washed with 100 ml of 1N-HCl, 100 ml of 5 % sodium carbonate aq. solution, 200 ml of water (twice) and 200ml of saturated brine. The obtained organic layer was then dried over anhydrous sodium sulfate, and the organic solvent was removed. The residue thus obtained was purified by a silica gel column chromatography, yielding 23.1 g of the subject compound as a colorless oil (Yield: 95%).
¹H-NMR (CDCl₃): δ 1.27 (3H, t, J = 7.2Hz), 1.36 (3H, d, J = 7.2Hz), 3.64 (1H, q, J = 7.2Hz), 3.88 (2H, s), 4.18 (2H, q, J = 7.2Hz), 6.95-7.15 (4H, m).
EI-MS:m/z = 238 (M+).

### (b) Synthesis of ethyl 4-(2-fluorophenyl)-3-hydroxy-2-methyl butyrate.

20.0 g (83.9 mmol) of ethyl 4-(2-fluorophenyl)-2-methyl-3 -oxobutyrate (see step (a) supra), 138 mg (0.084 mmol) of [{RuCl(((R)-SEGPHOS))₂ (µ-Cl)₃}[Me₂NH₂] and 80 ml of ethanol were placed into a stainless autoclave, and stirred for 18 hours at 80 °C under 3.0 MPa of hydrogen pressure. The solvent was then distilled off, and the residue formed was purified by a silica gel column chromatography method, to obtain 18.6 g of title compound as a colorless oily substance (Yield: 93%). The title compound was analysed by gas chromatography (hereafter referred to as GC), which indicated that the compound was a diastereomeric mixture of 1:1. The optical purities were 96.9% e.e. and 98.5% e.e., respectively.
The diastereomeric rate and the optical purity were determined by Chirasil DEX CB as a conventional method .
EI-MS:m/z = 241 (M+1)

### (c) Synthesis of 4-(2-fluorophenyl)-2-methylbutan-1,3 -diol.

Under nitrogen atmosphere was added 2.84 g (74.9 mmol) of lithium aluminum hydride to 30 ml of tetrahydrofuran (hereafter referred to as THF) to form a suspension. 18.0 g of ethyl 4-(2-fluorophenyl)-3-hydroxy-2-methylbutyrate (see step (b) supra) were dissolved in 180 ml of THF, and added drop-wise to the above suspension. After stirring for 1.5 hour at room temperature, 5 ml of water and 5 ml of 1N-NaOH aqueous solution were added successively thereto, to terminate the reaction. To this was added 100 ml of Et₂O, and the mixture was stirred further, to yield a suspension. The suspension thus obtained was filtered for purification. The filtrate was distilled off and the residue formed was purified by silica gel column chromatography method, to obtain 14.5 g of title compound as a colorless oily substance (Yield : 99%).
EI-MS m/z 199 (M+1)

### (d) Synthesis of 4-(2-fluorophenyl)-3-hydroxy-2-methylbutyl tosylate.

Under nitrogen atmosphere were cooled in ice bath a mixture of 13.5 g (68.1 mmol) of 4-(2-fluorophenyl)-2--methylbutan-1,3-diol (see step (c) supra), 14.2 ml (193 mmol) of triethylamine and 70 ml of dichloromethane. To this mixture were added 13.0 g (68.1mmol) of p-toluenesulfonyl chloride. After stirring overnight at room temperature, 100 ml of water was added to the mixture, and the whole solution was extracted 3 times with 200 ml each of dichloromethane. The combined organic layer was dried over anhydrous sodium sulfate and distilled off. The residue thus formed was purified through a silica gel column chromatography, to yield 21.1 g of title compound as a colorless oily substance (Yield: 88%).
EI-MS m/z 353 (M+1).

### (e) Synthesis of (+)-1-(2-fluorophenyl)-3-methylbutane-2-ol.

Under nitrogen atmosphere was added 2.28 g (60.2 mmol) of lithium aluminum hydride to 30ml of THF. To this mixture was added drop-wise a solution of 21.1 g (60.2 mmol) of 4-(2-fluorophenyl)-3-hydroxy-2-methylbutyl tosylate (see step (d) supra) dissolved in 210 ml of THF at room temperature. After stirring for 0.5 hour at room temperature, sodium sulfate 10 hydrates was slowly added until the effervescence was no longer formed. After further stirring, a suspension was obtained and filtered. The filtrate was distilled off and the residue formed was purified through a silica gel column chromatography, to obtain 9.6 g of title compound as a colorless solid substance (Yield: 88%). The optical purity was 98.1% e.e. according to the conventional GC method using Chirasil DEX CB.
¹H-NMR (CDCl₃): δ 1.01 (6H, d, J = 6.6Hz), 1.76 (1H, dqq, J = 5.5, 6.6, 6.6Hz), 2.63 (1H, dd, J = 9.3, 13.2Hz), 2.92 (1H, dd, J = 2.7, 13.2Hz), 3.64 (1H, m), 7.00-7.10 (2H, m), 7.18-7.28 (2H, m).
[α]_{D}²⁴: +27.9 (c 1.0, CHCl₃).
EI-MS:m/z = 182 (M+)

### (f) Synthesis of (+)-1-(2-fluorobenzyl)-2-methylpropyl mesylate (hereafter sometimes referred to as "MESYLATE").

Under nitrogen atmosphere was cooled in ice bath a mixture of 8.6 g (47.2 mmol) of (+)-1-(2-fluorophenyl) -3-methylbutane-2-ol (see step (e) supra), 7.9 ml (56.6mmol) of triethylamine and 40 ml of dichloromethane. To this were added drop-wise 4.0 ml (51.9 mmol) of methanesulfonyl chloride in ice bath. The ice bath was then removed and the mixture was stirred overnight at room temperature. 100 ml of water was added thereto, and the whole mixture was extracted 3 times with 100 ml each of dichloromethane. The combined organic layer was washed with 100 ml of saturated brine, and dried over anhydrous sodium sulfate. The solvent was then distilled off and the residue formed was purified by a silica gel column chromatography method, to obtain 12.5 g of title compound (Yield : 91%).
¹H-NMR (CDCl₃): δ 1.06 (3H, d, J = 7.0Hz), 1.08 (3H, d, J = 7.0Hz), 2.07 (1H, dqq, J = 4.2, 7.0, 7.0Hz), 2.48 (3H, s), 2.93 (1H, dd, J = 9.0, 14.6Hz), 3.06 (1H, dd, J = 4.4, 14.6Hz), 4.77 (1H, ddd, J = 4.2, 4.4, 9.0Hz), 6.98-7.15 (2H,m), 7.18-7.32 (2H,m).
[α]_{D}²⁴: +30.8 (c 1.0, CHCl₃).

### (g) Synthesis of (+)-1,2-bis(2-isopropyl-2,3-dihydro-1H-phosphindol-1-yl)benzene, referred to as (+)-iPr-BeePHOS.

Under nitrogen atmosphere was added 300 µl (2.32 mmol) of 1,2-bisphosphinobenzene to 9 ml of THF, and the mixture was cooled in ice bath. To this was added drop-wise 2.9 ml (4.64 mmol) of hexane solution containing 1.6M of n-butyllithium, and the mixture was stirred for 1 hour under cooling. 1.21 g (4.64 mmol) of MESYLATE (see step (f) supra) was dissolved in 12 ml of THF, and added drop-wise to the above mixture. The formed mixture was further stirred in ice bath for 1 hours, and the ice bath was removed. The mixture was then stirred for 1 hour at room temperature, and further cooled down in ice bath. To this, 4.4 ml (6.96 mmol) of hexane solution containing 1.6M of n-butyllithium was added drop-wise, and the ice bath was removed. The mixture was stirred overnight at room temperature, and 1 ml of water was added to the reaction mixture to terminate the reaction. The solvent was distilled off, and the residue formed was mixed with 10 ml of water The water layer was extracted twice with 20 ml each of Et₂O. The combined organic layer was washed with 10 ml of water, and the organic solvent was distilled off. The residue formed was purified by a silica gel column chromatography method, to obtain 227 mg of title compound (Yield : 23%).
m.p.: 70-71°C
¹H-NMR (CDCl₃): δ 1.05 (6H, d, J = 6.4Hz), 1.09 (6H, d, J = 6.8Hz), 2.09-2.21 (2H, m), 2.60-2.69 (2H, m), 2.93-3.01 (2H, m), 3.28-3.36 (2H, m), 6.74-6.80 (2H, m), 7.02-7.06 (2H, m), 7.18-7.40 (8H, m).
³¹P-NMR (CDCl₃): δ +0.20(s)
[α]_{D}²⁴: 186.1 (c 1.0, CHCl₃).
HRMS: 430.1960 (calcd.430.1978).

### Example 2 Synthesis of (+)-1-(2-diphenylphosphinophenyl)-2 -isopropyl-2,3-dihydro-1H-phosphindol-1-yl)benzene, referred to as (+)-m-iPr-BeePHOS.

Under nitrogen atmosphere, 1.0 g (3.40 mmol) of 2-(diphenylphosphino)phenylphosphine was added to 30 ml of THF, and the mixture was cooled in ice bath. To this was added drop-wise 2.1ml (3.40 mmol) of hexane solution containing 1.6M of n-butyllithium, and the mixture was stirred for 1 hour in ice bath. To this was added drop-wise 885 mg (3.40 mmol) of MESYLATE (see step (f) of Example 1 supra) dissolved in 10 ml of THF. The mixture was stirred for 1 hour in ice bath, and the ice bath removed. The mixture was further stirred for 1 hour at room temperature, and cooled down again in ice bath. To this, 3.2 ml (5.10 mmol) of hexane solution containing 1.6M of n-butyllithium was added drop-wise, and the ice bath was removed. The mixture was further stirred overnight at room temperature, and 1 ml of water was added to terminate the reaction. The solvent was distilled off, and the residue was mixed with 10 ml of water. The water layer was extracted 3 times with 20 ml each of Et₂O. The combined organic layer was washed with 10 ml of water and distilled off to obtain a residue. The residue obtained was purified by a silica gel column chromatography method, to obtain 400 mg of title compound (Yield : 27%).
m.p.: 41-42°C.
¹H-NMR (CDCl₃): δ 0.81 (3H, d, J = 6.6Hz), 0.83 (3H, d, J = 6.6Hz), 1.81 (1H, qqd, J = 6.6, 6.6, 8.8Hz), 2.20-2.28 (1H, m), 2.85 (1H, ddd, J = 4.9, 4.9, 16.5Hz), 3.24 (1H, ddd, J = 4.4, 8.2, 16.5Hz), 6.80-6.85 (1H, m), 6.93-6.98 (1H, m), 7.10-7.40 (3H, m), 7.20-7.40 (13H, m).
³¹P-NMR (CDCl₃) : δ -12.9(d,J=147.5Hz), -0.4(d,J=147.5Hz).
[α]_{D}²⁴: +84.7 (c 1.0, CHCl₃).
HRMS: 438.1597 (calcd. 438.1665).

### Example 3: Synthesis of (+)-1-(2-bis(3,5-di-tert-butyl-4-methoxy phenyl)phosphinophenyl)-2-isopropyl-2,3-dihydro-1H-phosphindo 1, referred to as (+)-DTBM-iPr-BeePHOS.

Under nitrogen atmosphere was added 1.0 g (1.72 mmol) of 2-(bis (3,5-di (tert-butyl) -4-methoxyphenyl) phosphino) phenyl phosphine to 30 ml of THF, and the mixture was cooled in ice bath. To this, 1.1ml (1.72 mmol) of hexane solution containing 1.6M of n-butyllithium was added drop-wise, and the mixture was stirred for 1 hour in ice bath. To this, 448 mg (1.72 mmol) of MESYLATE (see step (f) of Example 1) dissolved in 10 ml of THF was added drop-wise, and stirred for 1 hour in ice bath. After the ice bath was removed, the mixture was further stirred for 1 hour at room temperature. The mixture was again cooled in ice bath, and 1.6 ml (2.58 mmol) of hexane solution containing 1.6M of n-butyllithium was added drop-wise. After the ice bath was removed, the mixture was stirred overnight at room temperature, and 1 ml of water was added to terminate the reaction. The solvent was then distilled off, and the residue formed was mixed with 10 ml of water. The water layer was extracted 3 times with 20 ml each of Et₂O. The combined organic layer was washed with 10 ml of water, and the organic solvent was distilled off. The residue formed was purified by a silica gel column chromatography method, to obtain 337 mg of title compound (Yield : 27%).
m.p.: 69-70°C.
¹H-NMR (CDCl₃): δ 0.69 (3H, d, J = 6.6Hz), 0.71 (3H, d, J = 6.6Hz), 1.29-1.34 (36H, m), 1.63-1.73 (1H, m), 2.00-2.06 (1H, m), 2.78-2.86
(1H, m), 3.12-3.21 (1H, m), 3.67 (3H, s), 3.69 (3H, s), 6.75-6.80 (1H, m), 6.87-6.92 (1H, m), 7.02-7.30 (9H, m), .31-7.35 (1H, m).
³¹P-NMR(CDCl₃): δ -13.9 (d, J = 145.4Hz), -1.4 (d, J = 145.4Hz).
[α]_{D}²⁴: +8.45 (c 1.0, CHCl₃).
HRMS: 722.4397 (calcd. 722.4382).

### Example 4: Synthesis of (+)-1,2-bis(2-methyl-2,3-dihydro-1H -phosphindol-1-yl)benzene, referred to as (+)-Me-BeePHOS.

### (a) Synthesis of 2-(2-fluorophenyl)ethanol

Under nitrogen atmosphere, a solution of 10 g (64.9 mmol) of 2-fluorophenylacetic acid in 100 ml of THF was slowly added to a suspension of 2.46 g (64.0 mmol) of lithium aluminum hydride in 50 ml of THF at room temperature. After stirring for 1 hr, sodium sulfate 10 hydrates was slowly added until no effervescence was observed. The reaction mixture was filtrated and the obtained filtrate was distilled off to yield 8.83 g of title compound (Yield: 97 % ).
¹H NMR (CDCl₃): δ 1.48 (1H, bs), 2.93 (2H, t, J = 6.6 Hz), 3.87 (2H, td, J = 6.6, 6.6 Hz), 6.95-7.40 (4H, m);
EI-MS: m/z 140 (M)⁺

### (b) Synthesis of 2-(2-fluorophenyl)ethyl diisopropylcarbamate

Under nitrogen atmosphere, a mixture of 8.0 g (57.1 mmol) of 2-(2-fluorophenyl)ethanol, 9.34 g (57.1 mmol) of diisopropylcarbamoyl chloride and 6.93 ml (85.7 mmol) of pyridine was heated to 90 °C and stirred overnight. After cooling to room temperature, the mixture was treated with 1 N-HCl, and extracted 3 times with ethyl acetate. The ethyl acetate extracts were washed with water, a NaHCO₃ saturated aqueous solution, water and brine, and then dried over sodium sulfate. After the combined organic layer was distilled off, the obtained residue was purified through a silica gel column chromatography to give 14.0 g of title compound (Yield: 98 %).
¹H NMR (CDCl₃): δ 1.14 (12H, d, J = 6.8 Hz), 3.01 (2H, t, J = 6.8 Hz), 3.50-4.20 (2H, m), 4.31 (2H, t, J = 6.8 Hz), 6.95-7.30 (4H, m); EI-MS: m/z 267 (M)⁺

### (c) Synthesis of 2-(2-fluorophenyl)-1-methylethyl diisopropyl carbamate

Under nitrogen atmosphere, a solution of 14.1 g (60.2 mmol) of (-)-Sparteine in 75 ml of diethyl ether was cooled to -78 °C, and a 1.0-M solution of 60.2 ml (60.2 mmol) of s-butyllithium in cyclohexanes was added drop-wise thereto. The reaction mixture was stirred for 15 min and then added to a solution of 10 g (40.1 mmol) of 2-(2-fluorophenyl)ethyl diisopropylcarbamate dissolved in diethyl ether at -78 °C. After stirring for 4 hr, methyl iodide was added to the above mixture, and the whole solution was stirred for 2 hr. Water was then added to the solution and the resulting mixture was warmed to room temperature. The mixture was treated with 1 N-HCl, and extracted 3 times with ethyl acetate. The latter was washed with water and brine, and dried over sodium sulfate. The solvent was then distilled off and the obtained residue was purified on a silica gel column chromatography to yield 5.11 g of title compound (Yield: 45 %).
¹H NMR (CDCl₃): δ 1.16 (12H, d, J = 6.8 Hz), 1.25 (3H, d, J = 6.4 Hz), 2.80-3.10 (2H, m), 3.60-4.15 (2H, m), 5.12 (1H, tq, J = 6.4, 6.8 Hz), 6.90-7.30 (4H, m).

### (d) Synthesis of (+)-1-(2-fluorophenyl)propan-2-ol

Under nitrogen atmosphere, a 1.0-M solution of 182 ml (182 mmol) of diisobutylaluminum hydride in THF was added drop-wise to a solution of 5.11 g (18.2 mmol) of 2-(2-fluorophenyl)-1-methylethyl diisopropylcarbamate dissolved in THF. The reaction mixture was stirred overnight at room temperature, and sodium sulfate 10 hydrates was added slowly thereto until no effervescence was observed. After sodium sulfate was added, the mixture was filtrated. The filtrate was distilled off and the obtained residue was purified on a silica gel column chromatography, giving 2.15 g of title compound (Yield: 77 %; optical purity: 97% e.e.).
In the above example, the optical purity (% e.e.) was determined by GC (Chirasil DEX-CB).
[α]_{D}^{24:} +27.3 (c 1.0, CHCl₃);
¹H NMR (CDCl₃): δ 1.25 (3H, d, J = 6.2 Hz), 1.50 (1H, d, J = 4.4 Hz), 2.74 (1H, ddd, J = 1.0, 7.4. 13.4 Hz), 2.86 (1H, ddd, J = 1.2, 5.2, 13.4 Hz), 3.95-4.20 (1H, m), 6.95-7.35 (4H; m);
EI-MS: m/z 154 (M)⁺.

### (e) Synthesis of (+)-2-(2-fluorophenyl)-1-methylethyl mesyate

Under nitrogen atmosphere, 2.15 g (14.0 mmol) of (+)-1-(2-fluorophenyl)-propan-2-ol and 2.34 ml (16.8 mmol) of triethylamine in 10ml of dichloromethane were mixed, and 1.19 ml (15.4 mmol) of methanesulfonyl chloride was added to the mixture at 0 °C. The resulting mixture was stirred overnight at room temperature, treated with water and then extracted 3 times with dichloromethane. The combined organic layer was washed with water, and the solvent was distilled off. The obtained residue was purified on a silica gel column chromatography, giving 2.68 g of title compound (Yield: 82 %).
[α]_{D}²⁴: +23..4 (c 1.0, CHCl₃);
¹H NMR (CDCl₃): δ 1.48 (3H, d, J = 6.6 Hz), 2.62 (3H, s), 2.90-3.10 (2H, m), 4.95 (1H, qt, J = 6.6, 6.6 Hz), 6.95-7.40 (4H, m).

### (f) Synthesis of (+)-1,2-bis(2-methyl-2,3-dihydroxy-1H-phosphindol-1-yl) benzene, referred to as (+)-Me-BeePHOS.

1.80 g (7.74 mmol) of (+)-2-(2-fluorophenyl)-1-methylethyl mesylate was used instead of MESYLATE used in Example 1(g), and 367mg of title compound was obtained in a similar manner to that disclosed in Example 1(g) (Yield: 25%).
Mp: 148-150 °C;
[α]_{D}²⁴: +301.1 ( c 1.0, CHCl₃);
¹H NMR (CDCl₃): δ 1.42 (6H, dd, J = 7.7, 19.8 Hz), 2.74-2.81 (2H, m), 2.93 (2H, ddq, J = 2.7, 7.7, 7.7 Hz), 3.33-3.40 (2H, m), 6.53-6.60 (2H, m), 6.95-7.05 (2H, m), 7.26-7.32 (2H, m), 7.37-7.45 (2H, m), 7.56-7.60 (2H, m);
³¹P NMR (CDCl₃): δ 10.3;
HRMS: m/z 374.1321 (calc. 374.2353 for C₂₄H₂₄P₂).

### Example 5: Synthesis of (+)-1-(2-Diphenylphosphinophenyl) -2-methyl-2,3-dihydro-1H-phosphindole, referred to as (+)-m-BeePHOS.

The same procedure as Example 2 was used except that 738 mg (3.18 mmol) of (+)-2-(2-fluorophenyl)-1-methylethyl mesylate was used instead of MESYLATE, giving 686 mg of title compound (Yield: 53%).
Mp: 40-42 °C;
[α]_{D}²⁴: +55.1 (c 1.0, CHCl₃);
¹H NMR (CDCl₃): δ 1.06 (3H, dd, J = 7.1, 20.0 Hz), 2.10-2.30 (1H, m), 2.63 (1H, ddd, J = 3.0, 6.8, 16.2 Hz), 3.31 (1H, ddd, J = 2.4, 7.6, 16.2 Hz), 6.65-6.70 (1H, m), 6.98-7.03 (1H, m), 7.07-7.12 (1H, m), 7.15-7.19 (1H, m), 7.28-7.40 (13H, m), 7.49-7.53 (1H, m);
³¹P NMR (CDCl₃): δ -13.6 (d, J = 147.8 Hz), 7.7 (d, J = 147.8 Hz);
HRMS: m/z 410 (M)⁺.

### Example 6 Preapration of [Rh(cod)((+)-iPr-BeePHOS)]OTf

Under argon atmosphere, 54.3 mg (0.116 mmol) of [Rh(cod)₂]OTf was dissolved into 5 ml of dichloromethane in a 20 ml schlenk tube, and 2.5 ml of dichloromethane solution containing 50mg (0.116 mmol) of (+)-iPr-BeePHOS was added thereto at room temperature. After stirring overnight, the solvent was removed and the obtained residue was recrystallised in a solution of dichloromethane (0.5 ml)-diethyl ether (5 ml). The crystalline precipitates were filtered, washed twice with 5 ml each of diethyl ether and subjected to vacuum drying, to obtain 73.8 mg of title compound (Yield: 81 %).
¹H NMR (CD₂Cl₂): δ 1.18 (6H, d, J = 6.6 Hz), 1.22 (6H, d, J = 6.6 Hz), 2.17-2.36 (8H, m), 2.47-2.55 (2H, m), 2.88-2.99 (2H, m), 3.11-3.17 (2H, m), 3.68-3.78 (2H, m), 5.03-5.10 (2H, m), 5.14-5.20 (2H, m), 7.11-7.15 (2H, m), 7.32-7.41 (4H, m), 7.47-7.54 (4H, m), 7.58-7.63 (2H, m);
³¹P NMR (CD₂Cl₂): δ 73.4 (d, J = 151.5 Hz).

### Example 7: Preparation of [Rh(cod)((+)-m-iPr-BeePHOS)]OTf.

79.3 mg of title compound was obtained from 50 mg (0.114 mmol) of (+)-m-iPr-BeePHOS and 53.4 mg (0.114 mmol) of [Rh(cod)₂]OTf, according to a method similar to that used in Example 6 (Yield: 87%).
¹H NMR (CD₂Cl₂): δ 0.75 (3H, d, J = 6.6 Hz), 1.05 (3H, d, J = 6.6 Hz), 1.93-2.12 (3H, m), 2.17-2.34 (4H, m), 2.36-2.46 (2H, m), 2.86-2.95 (1H, m), 2.99-3.05 (1H, m), 3.59 (1H, ddd, J = 8.23, 16.5, 22.5 Hz), 4.83-4.93 (2H, m), 5.09-5.15 (1H, m), 5.21-5.28 (1H, m), 7.06-7.11 (1H, m), 7.28-7.36 (2H, m), 7.40-7.56 (12H, m), 7.56-7.65 (3H, m);
³¹P NMR (CD₂Cl₂): δ 60.8 (dd, J = 25.6, 151.5 Hz), 72.2 (dd, J = 25.6, 145.1 Hz).

### Example 8: Preparation of [Rh(cod)((+)-DTBM-iPr-BeePHOS)] OTf.

75.0 mg of title compound was obtained from 50 mg (0.069 mmol) of (+)-DTBM-iPr-BeePHOS and 32.4 mg (0.069 mmol) of [Rh(cod)₂]OTf according to a method similar to that used in Example 6, except that the purification was carried out by washing 3 times with 5 ml each of hexane, instead of recrystallisation in a solution of dichloromethane-diethylether (Yield: 100%).
¹H NMR (CD₂Cl₂): δ 0.85 (3H, d, J = 6.6 Hz), 1.11 (3H, d, J = 6.6 Hz), 1.36 (18H, s), 1.37 (18H, s), 2.03-2.20 (3H, m), 2.25-2.39 (4H, m), 2.43-2.50 (2H, m), 2.95-3.11 (2H, m), 3.65 (1H, ddd, J = 7.1, 15.9, 22.5 Hz), 4.87-4.94 (1H, m), 4.94-5.00 (1H, m), 5.11-5.18 (1H, m), 5.33-5.40 (1H, m), 7.10-7.14 (1H, m), 7.28-7.36 (3H, m), 7.40-7.46 (1H, m), 7.46-7.53 (4H, m), 7.55-7.64 (3H, m);
³¹P NMR (CD₂Cl₂): δ 62.0 (dd, J = 25.6, 149.4 Hz), 70.5 (dd, J = 25.6, 147.3 Hz).

### Example 9: Preparation of [Rh(cod)((+)-Me-BeePHOS)]OTf.

81 mg of title compound was obtained from 42 mg (0.114 mmol) of (+)-Me-BeePHOS and 53.4 mg (0.114 mmol) of [Rh(cod)₂]OTf, according to a method similar to that used in Example 6 (Yield: 98%).
¹H NMR (CD₂Cl₂): δ 1.52-1.60 (6H, m), 2.17-2.38 (6H, m), 2.48-2.57 (2H, m), 3.05-3.20 (4H, m), 3.65-3.75 (2H, m), 5.08-5.16 (2H, m), 5.16-5.23 (2H, m), 7.23-7.30 (2H, m), 7.36-7.44 (4H, m), 7.48-7.52 (2H, m), 7.52-7.59 (2H, m), 7.60-7.65 (2H, m);
³¹P NMR (CD₂Cl₂): δ 81.3 (d, J = 151.2 Hz).

### Example 10: Preparation of [Rh(cod)((+)-m-BeePHOS)]OTf.

85 mg of title compound was obtained from 46 mg (0.114 mmol) of (+)-m-BeePHOS and 53.4 mg (0.114 mmol) of [Rh(cod)₂]OTf, according to a method similar to that used in Example 6 (Yield: 98%).
¹H NMR (CD₂Cl₂): δ 1.30 (3H, dd, J = 7.1, 19.8 Hz), 2.04-2.60 (8H, m), 2.84-2.95 (1H, m), 2.98-3.03 (1H, m), 3.61 (1H, ddd, J = 8.2, 17.0, 18.7 Hz), 4.71-4.78 (1H, m), 4.90-4.98 (1H, m), 5.29-5.41 (2H, m), 7.22-7.27 (2H, m), 7.37-7.41 (2H, m), 7.43-7.75 (14H, m);
³¹P NMR (CD₂Cl₂): δ 60.5 (dd, J = 27.7, 151.5 Hz), 78.2 (dd, J = 27.7, 149.4 Hz).

### Example 11: Preparation of [RuCl(p-cymene)((+)-iPr-BeePHOS)] Cl.

Under argon atmosphere, 71.0 mg (0.116 mmol) of [RuCl₂(p-cymene)]₂ was dissolved into 2.5 ml of dichloromethane and 2.5ml of ethanol contained in a 20 ml Schlenk tube. To this, 100 mg (0.232 mmol) of (+)-iPr-BeePHOS was added and stirred overnight at 50 °C. After the stirring, the solvent was distilled off. The obtained residue was washed 3 times with 5ml each of diethylether and the residue after washing was subjected to vacuum drying, to yield 165 mg of title compound (Yield: 97%).
¹H NMR (CD₂Cl₂): δ 0.50 (3H, d, J = 6.6 Hz), 0.53 (3H, d, J = 6.6 Hz), 1.01 (3H, d, J = 6.6 Hz), 1.02 (3H, d, J = 6.6 Hz), 1.14 (3H, d, J = 7.1 Hz), 1.23 (3H, d, J = 7.1 Hz), 1.62 (3H, s), 2.06-2.17 (1H, m), 2.37 (1H, qq, J = 7.1, 7.1 Hz), 2.64-2.80 (2H, m), 3.00-3.08 (1H, m), 3.47-3.67 (3H, m), 3.76 (1H, ddd, J = 8.2, 10.4, 17.0 Hz), 5.90-5.95 (1H, m), 6.07-6.11 (1H, m), 6.12-6.17 (1H, m), 6.31-6.36 (1H, m), 7.10-7.16 (1H, m), 7.24-7.34 (2H, m), 7.34-7.40 (1H, m), 7.42-7.50 (2H, m), 7.52-7.60 (4H, m), 7.60-7.65 (1H, m), 7.76-7.81 (1H, m);
³¹P NMR (CD₂Cl₂): δ 87.1 (d, J = 36.3 Hz), 92.3 (d, J = 36.3 Hz).

### Example 12: Preparation of [RuCl(p-cymene)((+)-m-iPr -BeePHOS)]Cl.

80 mg of title compound was obtained from 46 mg (0.232 mmol) of (+)-m-iPr-BeePHOS with 71.0 mg (0.116 mmol) of [RuCl₂(p-cymene)]₂, according to a method similar to that used in Example 11 (Yield: 98%).
³¹P NMR (CD₂Cl₂): major δ 68.6 (d, J = 36.3 Hz), 82.3 (d, J = 36.3 Hz), minor δ 68.6 (d, J = 36.3 Hz), 84.3 (d, J = 36.3 Hz).

### Example 13: Preparation of [RuCl(p-cymene)((+)-Me-BeePHOS)] Cl.

71 mg of title compound was obtained from 87 mg (0.232 mmol) of (+)-Me-BeePHOS and 71.0 mg (0.116 mmol) of [RuCl₂(p-cymene)]₂, according to a method similar to that used in Example 11 (Yield: 97%).
¹H NMR (CD₂Cl₂): δ 0.76 (3H, d, J = 7.1 Hz), 0.89 (3H, d, J = 7.1 Hz), 1.49 (3H, dd, J = 7.1, 18.7 Hz), 1.50 (3H, s), 1.64 (3H, dd, J = 7.1, 19.8 Hz), 2.21 (1H, qq, J = 7.1, 7.1 Hz), 3.07-3.19 (1H, m), 3.19-3.27 (1H, m), 3.33 (1H, ddd, J = 3.8, 10.4, 17.6 Hz), 3.56-3.70 (2H, m), 4.05 (1H, ddd, J = 7.7, 7.7, 17.6 Hz), 5.75-5.81 (1H, m), 5.94-6.00 (1H, m), 6.31-6.37 (1H, m), 6.49-6.57 (1H, m), 7.21-7.31 (2H, m), 7.32-7.41 (2H, m), 7.42-7.53 (3H, m), 7.53-7.61 (4H, m), 7.78-7.85 (1H, m);
³¹P NMR (CD₂Cl₂): δ 86.3 (d, J = 36.3 Hz), 95.1 (d, J = 36.3 Hz).

### Example 14: Preparation of [RuCl(p-cymene)((+)-m-BeePHOS)] Cl.

77 mg of title compound was obtained from 95 mg (0.232 mmol) of (+)-m-BeePHOS and 71.0 mg (0.116 mmol) of [RuCl₂(p-cymene)]₂, according to a method similar to that used in Example 11 (Yield: 98%).
³¹P NMR (CD₂Cl₂): major δ 67.7 (d), 88.8 (d), minor δ 66.9 (d), 81.9 (d).

### Example 15: Praparation of Ru(OAc)₂((+)-iPr-BeePHOS).

Under argon atmosphere, 100 mg (0.136 mmol) of [RuCl(p-cymene)(+)-iPr-BeePHOS]Cl and 27.9 mg (0.34 mmol) of sodium acetate were dissolved into 5ml of dioxane contained in a 20ml Schlenk tube . The mixture was stirred overnight at 100 °C, cooled to room temperature and filtered. After the filtration, the solvent was distilled off and the obtained residue was subjected to vacuum drying, to yield 63.6 mg of title compound (Yield: 72 %).
¹H NMR (CD₂Cl₂): δ 0.51 (6H, d, J = 6.6 Hz), 1.07 (6H, d, J = 7.1 Hz), 1.10 (6H, s), 2.08-2.19 (2H, m), 2.93-3.02 (2H, m), 3.34-3.39 (2H, m), 3.43-3.52 (2H, m), 6.75-6.78 (2H, m), 7.07-7.10 (2H, m), 7.35-7.40 (4H, m), 7.47-7.50 (2H, m), 7.67-7.72 (2H, m);
³¹P NMR (CD₂Cl₂): δ 111.7.

### Example 16: Preparation of Ru(OAc)₂((+)-m-iPr-BeePHOS).

60 mg of title compound was obtained from 101.7 mg (0.232 mmol) of (+)-m-BeePHOS with 82.2 mg (0.116 mmol) of [RuCl(p-cymene)((+)-m-iPr-BeePHOS)]Cl, according to a method similar to that used in Example 15 (Yield: 80%).
¹H NMR (CD₂Cl₂): δ 0.34 (3H, d, J = 6.0 Hz), 1.03 (3H, J = 6.6 Hz), 2.96-3.05 (1H, m), 3.28-3.34 (1H, m), 3.41-3.52 (1H, m), 6.48-6.51 (1H, m), 6.99-7.05 (1H, m), 7.22-7.52 (12H, m), 7.58-7.68 (2H, m), 7.68-7.74 (1H, m), 7.74-7.80 (1H, m);
³¹P NMR (CD₂Cl₂): δ 92.4 (d, J = 32.3 Hz), 111.2 (m).

### Use Example

Asymmetric hydrogenation of methyl N-acetamido cinnamate.

A mixture of 2.3 mg (0.0029 mmol) of [Rh(cod)(+)-iPr-BeePHOS]OTf, 125 mg (0.570 mmol) of methyl N-acetamidocinnamate and 1.5 ml of methanol were placed into a stainless autoclave The mixture was slightly heated for 15 hours with stirring, under 0.4 MPa of hydrogen pressure at 30 °C. The enantiomeric excesses (% e.e.) and conversions (%) were measured by HPLC directly, to give a yield of, respectively, 97.9 % e.e. and >99 %.
The HPLC used had the following features: Daicel Chiralcel OJ, hexane : 2-propanol = 90 : 10 (1.0 ml/min), 254 nm.

## Claims

1. A phosphine compound of the formula (1): 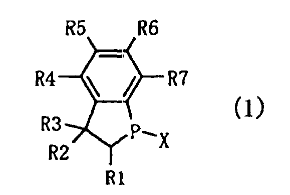
wherein R¹ is a linear or branched alkyl group having 1 to 5 carbon atoms or a cyclohexyl group;
R² and R³ each represent independently a hydrogen atom or an alkyl group having 1 to 5 carbon atoms;
R⁴ , R⁵ , R⁶ and R⁷ each represent independently a hydrogen atom, a halogen atom, an alkyl group having 1 to 5 carbon atoms, an alkoxy group having 1 to 5 carbon atoms, or a di-alkyl amino group, each alkyl group having 1 to 5 carbon atoms;
X represents a substituted or non-substituted alkyl group, aryl group, aralkyl group, heterocyclic group and alkoxy group, each having 1 to 36 carbon atoms, and an amino group and derivatives thereof; and
wherein a ring or condensed ring may or may not be formed between the members of at least one pair selected from the group consisting of R⁴ and R⁵, R⁵ and R⁶ and R⁶ and R⁷.

2. A phosphine compound of the formula (2): 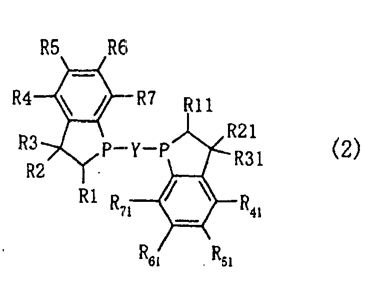
wherein R¹ to R⁷ are as defined in claim 1;
R¹¹ is a linear or branched alkyl group having 1 to 5 carbon atoms or a cyclohexyl group;
R²¹ and R³¹ each represent independently a hydrogen atom or an alkyl group having 1 to 5 carbon atoms;
R⁴¹, R⁵¹, R⁶¹ and R⁷¹ each represent independently a hydrogen atom, a halogen atom, an alkyl group having 1 to 5 carbon atoms, an alkoxy group having 1 to 5 carbon atoms, or a di-alkyl amino group, each alkyl group having 1 to 5 carbon atoms;
Y represents a functional group that may form a stable bond with phosphorous atom; and
wherein a ring or condensed ring may or may not be formed between the members of at least one pair selected from the group consisting of R⁴¹ and R⁵¹, R⁵¹ and R⁶¹, and R⁶¹ and R⁷¹.

3. A phosphine compound of the formula (2) according to claim 2, wherein R¹ and R¹¹ represent an isopropyl group, respectively, and Y represents 1,2-phenylene.

4. A transition metal complex comprising a transition metal and, as ligand, a compound defined in any one of claims 1 to 3.

5. A transition metal complex according to claim 4, **characterized in that** said transition metal comprises at least one metal selected from the group consisting of rhodium, ruthenium, iridium, palladium and nickel.

6. A catalyst for asymmetric syntheses comprising the transition metal complex according to claim 4 or 5.

7. A catalyst according to claim 6, **characterized in that** said asymmetric syntheses comprise the asymmetric hydrogenation.

8. A method for preparing a phosphine compound according to claim 1 or 2, comprising the step of:
a) reducing 2-halophenylacetic acid using a reducing agent, thereby obtaining 2-(2-halophenyl)ethanol;
b) reacting said 2-(2-halophenyl)ethanol with dialkylcarbamoyl halide in the presence of an amine, thereby obtaining a corresponding carbamate;
c) reacting said carbamate with a reagent which comprises alkyllithium and optically active diamine, then with an alkylating agent, thereby obtaining an alkylated carbamate;
d) reducing said alkylated carbamate, thereby obtaining an optically active secondary alcohol;
e) mesylating or tosylating the hydroxyl group of said secondary alcohol, thereby obtaining a corresponding mesylate or tosylate; and
f) reacting said corresponding mesylate or tosylate successively with a base compound, a functional group that may have a stable bond with a phosphorous atom, and a base compound.

9. A method for preparing a phosphine compound according to claim 3, comprising the step of:
a) reacting 2-halogenophenylacetic acid successively with 1,1'-carbonyl diimidazole and alkyl alkylmalonate mono alkaline salt in the presence of a metal halide catalyst, thereby obtaining alkyl 4-(2-halogenophenyl)-2-alkyl-3-oxobutyrate;
b) performing an asymmetric hydrogenation of said alkyl 4-(2-halogenophenyl)-2-alkyl-3-oxobutyrate in the presence of an optically active diphosphine-Ru complex, thereby obtaining an optically active alkyl 4-(2-halogenophenyl)-3-hydroxy-2 -alkylbutyrate;
c) reducing said alkylbutyrate with a reducing agent, thereby obtaining a diol;
d) tosylating or mesylating the primary hydroxyl group of said diol, thereby obtaining a tosylate or mesylate;
e) reducing said tosylate or mesylate with a reducing agent, thereby obtaining an optically active 1-(2-halogenophenyl) -3-alkylbutan-2-ol;
f) mesylating or tosylating said alcohol, thereby obtaining a mesylate or tosylate thereof; and
reacting said mesylate or tosylate successively with a base compound, 1,2-bisphosphinobenzene and a base compound.

10. A use of a phosphine compound according to any one of claims 1to 3, for preparing a catalyst for asymmetric syntheses.

## Patentansprüche

1. Phosphin-Verbindung der Formel (1): 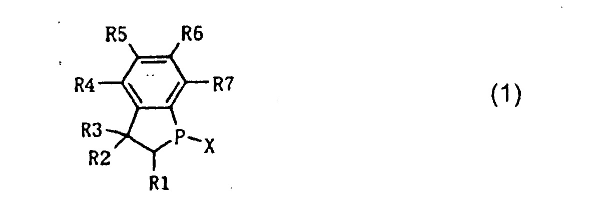
wobei R¹ eine lineare oder verzweigte Alkylgruppe mit 1 bis 5 Kohlenstoffatomen oder eine Cyclohexylgruppe ist;
R² und R³ jeweils unabhängig ein Wasserstoffatom oder eine Alkylgruppe mit 1 bis 5 Kohlenstoffatomen darstellt;
R⁴, R⁵, R⁶ und R⁷ jeweils unabhängig ein Wasserstoffatom, ein Halogenatom, eine Alkylgruppe mit 1 bis 5 Kohlenstoffatomen, eine Alkoxygruppe mit 1 bis 5 Kohlenstoffatomen, oder eine Dialkylamino-Gruppe, wobei jede Alkylgruppe 1 bis 5 Kohlenstoffatome aufweist, darstellt;
X eine substituierte oder unsubstituierte Alkylgruppe, Arylgruppe, Aralkylgruppe, heterocylische Gruppe und Alkoxygruppe, wobei jede 1 bis 36 Kohlenstoffatome aufweist, und eine Aminogruppe und Derivate davon darstellt; und
wobei ein Ring oder kondensierter Ring zwischen den Mitgliedern von mindestens einem Paar, ausgewählt aus der Gruppe bestehend aus R⁴ und R⁵, R⁵ und R⁶, und R⁶ und R⁷, gebildet oder nicht gebildet werden kann.

2. Phosphin-Verbindung der Formel (2): 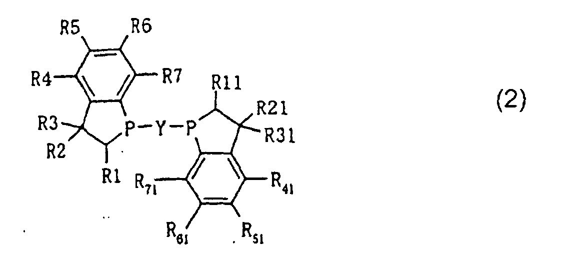
wobei R¹ bis R⁷ wie in Anspruch 1 definiert sind;
R¹¹ eine lineare oder verzweigte Alkylgruppe mit 1 bis 5 Kohlenstoffatomen oder eine Cyclohexylgruppe ist;
R²¹ und R³¹ jeweils unabhängig ein Wasserstoffatom oder eine Alkylgruppe mit 1 bis 5 Kohlenstoffatomen darstellt;
R⁴¹, R⁵¹, R⁶¹ und R⁷¹ jeweils unabhängig ein Wasserstoffatom, ein Halogenatom, eine Alkylgruppe mit 1 bis 5 Kohlenstoffatomen, eine Alkoxygruppe mit 1 bis 5 Kohlenstoffatomen, oder eine Dialkylamino-Gruppe, wobei jede Alkylgruppe 1 bis 5 Kohlenstoffatome aufweist.
Y eine funktionelle Gruppe darstellt, die eine stabile Bindung mit einem Phosphoratom bilden kann; und
wobei ein Ring oder kondensierter Ring zwischen den Mitgliedern von mindestens einem Paar, ausgewählt aus der Gruppe bestehend aus R⁴¹ und R⁵¹, R⁵¹ und R⁶¹, und R⁶¹ und R⁷¹, gebildet oder nicht gebildet werden kann.

3. Phosphin-Verbindung der Formel (2) nach Anspruch 2, wobei R¹ bzw. R¹¹ eine Isopropylgruppe darstellt und Y 1,2-Phenylen darstellt.

4. Übergangsmetall-Komplex, umfassend ein Übergangsmetall und, als Ligand, eine Verbindung definiert wie in einem der Ansprüche 1 bis 3.

5. Übergangsmetall-Komplex nach Anspruch 4, wobei der Übergangsmetall-Komplex mindestens ein Metall, ausgewählt aus der Gruppe, bestehend aus Rhodium, Ruthenium, lridium, Palladium und Nickel, umfasst.

6. Katalysator für asymmetrische Synthesen, umfassend den Übergangsmetall-Komplex nach Anspruch 4 oder 5.

7. Katalysator nach Anspruch 6, wobei die asymmetrischen Synthesen die asymmetrische Hydrogenierung umfassen.

8. Verfahren zur Herstellung einer Phosphin-Verbindung nach Anspruch 1 oder 2, umfassend den Schritt
a) des Reduzierens von 2-Halophenylessigsäure unter Verwendung eines Reduktionsmittels, wobei 2-(2-Halophenyl)ethanol erhalten wird;
b) des Umsetzens des 2-(2-Halophenyl)ethanols mit Dialkylcarbamoyl-Halogenid in der Gegenwart eines Amins, wobei ein korrespondierendes Carbamat erhalten wird;
c) des Umsetzens des Carbamats mit einem Reagenz, umfassend Alkyllithium und optisch aktives Diamin, dann mit einem Alkylierungsmittel, wobei ein alkyliertes Carbamat erhalten wird;
d) des Reduzierens des alkylierten Carbamats, wobei ein optisch aktiver sekundärer Alkohol erhalten wird;
e) des Mesylierens oder Tosylierens der Hydroxylgruppe des sekundären Alkohols, wobei ein korrespondierendes Mesylat oder Tosylat erhalten wird; und
f) des Umsetzens des korrespondierenden Mesylats oder Tosylats nacheinander mit einer Basen-Verbindung, einer funktionellen Gruppe, die eine stabile Bindung mit einem Phosphoratom aufweisen kann, und einer Basen-Verbindung.

9. Verfahren zur Herstellung einer Phosphin-Verbindung nach Anspruch 3, umfassend den Schritt
a) des Umsetzens von 2-Halogenphenylessigsäure nacheinander mit 1,1'-Carbonyldiimidazol und Alkyl-Alkylmalonat-Monoalkalisalz in Gegenwart eines Metallhalogenid-Katalysators, wobei Alkyl-4-(2-halogenphenyl)-2-alkyl-3-oxobutyrat erhalten wird;
b) des Durchführens einer asymmetrischen Hydrogenierung des Alkyl-4-(2-halogenphenyl)-2-alkyl-3-oxobutyrats in Gegenwart eines optisch aktiven Diphosphin-Ru-Komplexes, wobei ein optisch aktives Alkyl-4-(2-halogenphenyl)-3-hydroxy-2-alkylbutyrat erhalten wird;
c) des Reduzierens des Alkylbutyrats mit einem Reduktionsmittel, wobei ein Diol erhalten wird;
d) des Tosylierens oder Mesylierens der primären Hydroxylgruppe des Diols, wobei ein Tosylat oder Mesylat erhalten wird;
e) des Reduzierens des Tosylats oder Mesylats mit einem Reduktionsmittel, wobei ein optisch aktives 1-(2-halogenphenyl)-3-alkylbutan-2-ol erhalten wird;
f) des Mesylierens oder Tosylierens des Alkohols, wobei ein Mesylat oder Tosylat davon erhalten wird; und
des Umsetzens des Mesylats oder Tosylats nacheinander mit einer Basen-Verbindung, 1,1-Bisphosphinobenzol und einer Basen-Verbindung.

10. Verwendung einer Phosphin-Verbindung nach einem der Ansprüche 1 bis 3, zur Herstellung eines Katalysators für asymmetrische Synthesen.

## Revendications

1. Phosphine de formule (1) : 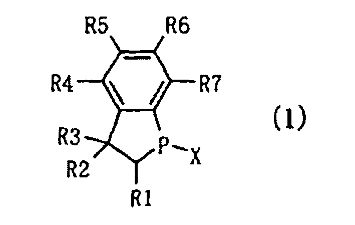
dans laquelle R¹ est un groupe alkyle à chaîne droite ou ramifiée ayant 1 à 5 atomes de carbone, ou un groupe cyclohexyle ;
R² et R³ représentent chacun indépendamment de l'autre un atome d'hydrogène ou un groupe alkyle ayant 1 à 5 atomes de carbone ;
R⁴, R⁵, R⁶ et R⁷ représentent chacun indépendamment des autres un atome d'hydrogène, un atome d'halogène, un groupe alkyle ayant 1 à 5 atomes de carbone, un groupe alcoxy ayant 1 à 5 atomes de carbone ou un groupe dialkylamino, chaque groupe alkyle ayant 1 à 5 atomes de carbone ;
X représente un groupe alkyle substitué ou non substitué, un groupe aryle, un groupe aralkyle, un groupe hétérocyclique et un groupe alcoxy, ayant chacun 1 à 36 atomes de carbone, et un dérivé aminé, et leurs dérivés ; et
où un noyau ou un noyau condensé peut, ou non, être formé entre les membres d'au moins une paire choisie dans l'ensemble consistant en R⁴ et R⁵, R⁵ et R⁶, et R⁶ et R⁷.

2. Phosphine de formule (2) : 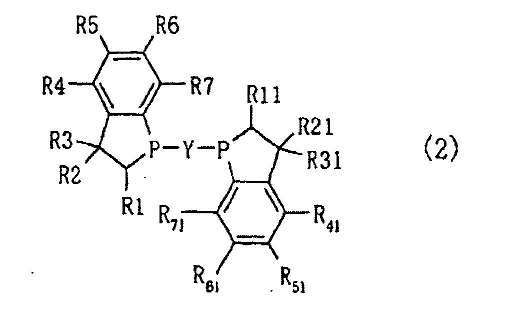
dans laquelle R¹ à R⁷ sont tels que définis dans la revendication 1 ;
R¹¹ est un groupe alkyle à chaîne droite ou ramifiée ayant 1 à 5 atomes de carbone, ou un groupe cyclohexyle ;
R²¹ et R³¹ représentent chacun indépendamment de l'autre un atome d'hydrogène ou un groupe alkyle ayant 1 à 5 atomes de carbone ;
R⁴¹, R⁵¹, R⁶¹ et R⁷¹ représentent chacun indépendamment des autres un atome d'hydrogène, un atome d'halogène, un groupe alkyle ayant 1 à 5 atomes de carbone, un groupe alcoxy ayant 1 à 5 atomes de carbone ou un groupe dialkylamino, chaque groupe alkyle ayant 1 à 5 atomes de carbone ;
Y représente un groupe fonctionnel pouvant former une liaison stable avec un atome de phosphore ; et
où un noyau ou un noyau condensé peut, ou non, être formé entre les membres d'au moins une paire choisie dans l'ensemble consistant en R⁴¹ et R⁵¹, R⁵¹ et R⁶¹, et R⁶¹ et R⁷¹.

3. Phosphine de formule (2) selon la revendication 2, dans laquelle R¹ et R¹¹ représentent chacun un groupe isopropyle, et Y est le radical 1,2-phénylène.

4. Complexe d'un métal de transition comprenant un métal de transition et, en tant que ligand, un composé selon l'une quelconque des revendications 1 à 3.

5. Complexe d'un métal de transition selon la revendication 4, **caractérisé en ce que** ledit métal de transition comprend au moins un métal choisi dans l'ensemble consistant en le rhodium, le ruthénium, l'iridium, le palladium et le nickel.

6. Catalyseur de synthèses asymétriques, comprenant le complexe d'un métal de transition selon la revendication 4 ou 5.

7. Catalyseur selon la revendication 6, **caractérisé en ce que** lesdites synthèses asymétriques comprennent l'hydrogénation asymétrique.

8. Procédé de préparation d'une phosphine selon la revendication 1 ou 2, comprenant les étapes consistant :
a) à réduire un acide 2-halogophénylacétique par utilisation d'un agent réducteur pour obtenir un 2-(2-halogénophényl)éthanol ;
b) à faire réagir ledit 2-(2-halogénophényl)éthanol avec un halogénure de dialkylcarbamoyle en présence d'une amine, pour obtenir un carbamate correspondant ;
c) à faire réagir ledit carbamate avec un réactif qui comprend un alkyllithium et une diamine optiquement active, puis avec un agent d'alkylation pour obtenir un carbamate alkylé ;
d) à réduire ledit carbamate alkylé pour obtenir un alcool secondaire optiquement actif ;
e) à mésyler ou tosyler le groupe hydroxyle dudit alcool secondaire pour obtenir un mésylate ou un tosylate correspondant ; et
f) à faire réagir successivement ledit mésylate ou tosylate correspondant avec un composé basique, un composé fonctionnel pouvant avoir une liaison stable avec un atome de phosphore, et un composé basique.

9. Procédé de préparation d'une phosphine selon la revendication 3, comprenant les étapes consistant :
a) à faire réagir un acide 2-halogénophénylacétique successivement avec du 1,1'-carbonyldiimidazole et un sel alkylmalonate d'alkyle et de mono(métal alcalin) en présence d'un catalyseur à base d'un halogénure métallique, pour obtenir un 4-(2-halogénophényl)-2-alkyl-3-oxobutyrate d'alkyle ;
b) à mettre en oeuvre une hydrogénation asymétrique dudit 4-(2-halogénophényl)-2-alkyl-3-oxobutyrate d'alkyle en présence d'un complexe diphosphine-Ru optiquement actif, pour obtenir un 4-(2-halogénophényl)-3-hydroxy-2-alkylbutyrate d'alkyle optiquement actif ;
c) à réduire ledit alkylbutyrate à l'aide d'un agent réducteur, pour obtenir un diol ;
d) à tosyler ou mésyler le groupe hydroxyle primaire dudit diol, pour obtenir un tosylate ou un mésylate ;
e) à réduire ledit tosylate ou mésylate avec un agent réducteur, pour obtenir un 1-(2-halogénophényl)-3-alkylbutane-2-ol optiquement actif ;
f) à mésyler ou tosyler ledit alcool, pour obtenir un mésylate ou tosylate de ce dernier ; et
à faire réagir ledit mésylate ou tosylate successivement avec un composé basique, du 1,2-bisphosphinobenzène et un composé basique.

10. Utilisation d'une phosphine selon l'une quelconque des revendications 1 à 3 pour préparer un catalyseur destiné à des synthèses asymétriques.
